# EUROPÄISCHE PATENTANMELDUNG

(11) **EP 1 591 060 A1**
(43) Veröffentlichungstag der Anmeldung: **02.11.2005**
(21) Anmeldenummer: 04405266.0
(22) Anmeldetag: 29.04.2004
(51) Int. Cl.: A61B 3/16

(54) **Schutzüberzug für ophthalmologische Tonometer und Herstellungsverfahren**

(71) Anmelder: SMT Swiss Microtechnology AG, 2562 Port (CH)
(72) Erfinder: Kanngiesser,Hartmut, 8038 Zürich (CH)
(74) Vertreter: Vogel, Dany

(57) **Zusammenfassung**

Es werden ein elastischer Schutzüberzug (1) für ophthalmologische Tonometer, welche eine Kontaktfläche zum Aufsetzen auf ein Auge aufweisen, sowie ein Verfahren zur einstückigen Herstellung des Schutzüberzugs (1) vorgeschlagen. Der Schutzüberzug (1) weist eine elastische Membran (14) mit einer Membrandicke von weniger als 55µm zum Abdecken der Kontaktfläche auf. Bei der Herstellung des Schutzüberzugs (1) wird in einem ersten Schritt elastisches Material in einen Hohlraum zwischen einem äusseren Formteil und einem inneren Formteil gespritzt. In einem zweiten Schritt wird ein Teil des Schutzüberzugs (1) als Membran (14) zum Abdecken der Kontaktfläche gebildet, indem das innere Formteil und das äussere Formteil im Bereich der zu bildenden Membran (14) aufeinander zu bewegt werden, so dass eine Membran (14) mit einer konstanten Membrandicke im Bereich von 30µm bis 50µm gebildet wird. Das Herstellungsverfahren ermöglicht eine kostengünstige einstückige Herstellung von elastischen Schutzüberzügen (1) mit dünnen Membranen (14), welche Augendruckmessungen nicht oder nur geringfügig beeinträchtigen.

## Beschreibung

### Technisches Gebiet

Die vorliegende Erfindung betrifft einen Schutzüberzug für ophthalmologische Tonometer und ein Verfahren zum Herstellen des Schutzüberzugs. Die Erfindung betrifft insbesondere einen Schutzüberzug für ophthalmologische Tonometer, welche eine Kontaktfläche zum Aufsetzen auf ein Auge aufweisen, und ein Verfahren zum Herstellen des Schutzüberzugs, wobei in einem ersten Schritt elastisches Material in einen Hohlraum zwischen einem äusseren Formteil und einem inneren Formteil gespritzt wird.

### Stand der Technik

In der Ophthalmologie werden Tonometer zum Messen des Augendrucks verwendet. Bei Tonometern, die eine Kontaktfläche zum Aufsetzen auf das Auge aufweisen, kann es bei der Berührung des Auges durch die Kontaktfläche zur Übertragung von Tränenflüssigkeit und damit auch von Krankheitserregern von einem Patienten zu einem anderen kommen. Zudem können Keime oder Verunreinigungen, die an der Kontaktfläche haften, bei der Messung ins Auge gebracht werden. Um dies zu vermeiden, wird das Tonometer, insbesondere dessen Kontaktfläche, entsprechend gereinigt oder mit einem sauberen möglichst sterilen Schutzüberzug versehen. Anlässlich von Augenuntersuchungen werden bei fast jedem Patienten Augendruckmessungen vorgenommen, wobei die Messungen im Allgemeinen jeweils bloss wenige Sekunden dauern. Die Vorbereitung der Tonometer, insbesondere deren Messköpfe und Kontaktflächen, für die Messung sollte folglich auch möglichst schnell zu bewerkstelligen sein. Ansonsten muss der behandelnde Arzt oder Optiker mehrere Tonometer oder Messköpfe zur Verfügung haben, die er wechselweise verwendet. In der Regel werden wegwerfbare Schutzüberzüge verwendet, die für den einmaligen Gebrauch bestimmt sind. Angesichts der grossen Anzahl von Augendruckmessungen und der dabei verwendeten Schutzüberzüge sollten die Schutzüberzüge möglichst kostengünstig herstellbar sein. Überdies sollten die Schutzüberzüge so beschaffen sein, dass sie die Augendruckmessung möglichst geringfügig oder gar nicht beinträchtigen. Insbesondere für Tonometer die Drucksensoren umfassen, beispielsweise ein Tonometer nach EP 1250884, sollten die Schutzüberzüge zum Abdecken der Kontaktfläche und des darin eingelassenen Drucksensors, Membranen mit Wandstärken aufweisen, die die Druckmessung nicht beeinträchtigen. In herkömmlichen Spritzgussverfahren, konnten bestenfalls Schutzüberzüge mit Membranen hergestellt werden, die eine Wandstärke von 70µm aufweisen, wobei der Anguss dazu zentral im Membranbereich vorgenommen werden musste. Sowohl Membranen mit Wandstärken von 70µm als auch zentrale Angussstellen im Membranbereich beeinträchtigen die Augendruckmessung durch Tonometer mit Drucksensoren in einem wesentlichen Ausmass.

### Darstellung der Erfindung

Es ist eine Aufgabe der vorliegenden Erfindung, einen neuen Schutzüberzug für ophthalmologische Tonometer und ein neues Verfahren zum Herstellen des Schutzüberzugs vorzuschlagen. Es ist insbesondere eine Aufgabe der vorliegenden Erfindung, einen Schutzüberzug für ophthalmologische Tonometer, die eine Kontaktfläche zum Aufsetzen auf ein Auge aufweisen, und ein Verfahren zum Herstellen des Schutzüberzugs vorzuschlagen, wobei der Schutzüberzug möglichst kostengünstig herstellbar sein soll und die Augendruckmessung durch den Schutzüberzug möglichst geringfügig oder gar nicht beeinträchtigt werden soll.

Gemäss der vorliegenden Erfindung werden diese Ziele insbesondere durch die Elemente der unabhängigen Ansprüche erreicht. Weitere vorteilhafte Ausführungsformen gehen ausserdem aus den abhängigen Ansprüchen und der Beschreibung hervor.

Die oben genannten Ziele werden durch die vorliegende Erfindung insbesondere dadurch erreicht, dass der Schutzüberzug für ophthalmologische Tonometer, welche eine Kontaktfläche zum Aufsetzen auf ein Auge aufweisen, zum Abdecken der Kontaktfläche eine elastische Membran mit einer Membrandicke von weniger als 55µm aufweist, vorzugsweise eine konstante Membrandicke im Bereiche von 30µm bis 50µm, und dass der Schutzüberzug einstückig hergestellt wird. Durch die einstückige Herstellung eines Schutzüberzugs mit einer derart dünnen Membran entfallen einerseits technisch aufwendige Verfahrensschritte zum Zusammenfügen der Membran mit einem oder mehreren Trägerteilen und andererseits werden die mittels eines Drucksensors des Tonometers durchgeführten Messungen durch die Membran nur geringfügig oder gar nicht beeinträchtigt.

Vorzugsweise weist der Schutzüberzug eine Öffnung und einen die Öffnung umschliessenden Mantel auf. Der Mantel bildet einen sich von der Öffnung zur Membran konisch verjüngenden Innenraum und weist einen dem Innenraum zugewandten Haftbereich auf. Diese Ausgestaltung des Schutzüberzugs ermöglicht das Einführen einer sich konisch verjüngenden (im wesentlichen kegelstumpfförmigen) Messkopfspitze des Tonometers durch die Öffnung und die Aufnahme der Messkopfsspitze im Innenraum, wobei die Kontaktfläche durch die Membran abgedeckt wird. Der dem Innenraum zugewandte Haftbereich bewirkt, dass der Schutzüberzug im angebrachten Zustand an der Messkopfspitze des Tonometers haftet.

Vorzugsweise ist der Mantel aus einem Elastomer, beispielsweise aus Silikon, hergestellt, und der Haftbereich weist eine glatte Oberfläche auf und ist in einem die Öffnung umlaufenden Bereich angeordnet. Die Verwendung eines Elastomers für den Mantel verleiht dem Mantel einerseits Dehnbarkeit und Elastizität und andererseits kann durch Strukturierung der Oberfläche des Schutzüberzugs deren Haftwirkung kontrolliert werden. Eine mit Erosionsstruktur versehene, aufgerauhte Oberfläche weist eine kleine Haftwirkung auf, während eine polierte, glatte, Oberfläche eine grosse Haftwirkung aufweist. Dadurch, dass der Haftbereich im Bereich angeordnet ist, der dem Innenraum zugewandt ist und die Öffnung umläuft, bleibt der Schutzüberzug im Randbereich, der dem Innenraum zugewandt ist und an die Öffnung angrenzt, an der eingeführten Messkopfspitze haften. Dabei kann der elastische Mantel leicht gestreckt werden und so eine Spannwirkung auf die Membran übertragen.

Vorzugsweise ist die Membran aus einem Elastomer, beispielsweise aus Silikon, hergestellt, und weist eine glatte Oberfläche auf, welche im auf der Messkopfspitze angebrachten Zustand des Schutzüberzugs auf der Kontaktfläche haftet. Durch die glatte Oberfläche wird der aus einem Elastomer hergestellten Membran wie oben angeführt eine Haftwirkung verliehen. Wenn beim Anbringen des Schutzüberzugs beispielsweise mittels eines Stempels Luft, die zwischen der Membran und der Kontaktfläche eingeschlossen ist, aus dem Bereich zwischen der Membran und der Kontaktfläche verdrängt wird, kann durch das Anhaften der Membran an der Kontaktfläche und durch die oben erwähnte Spannkraft das Wiedereintreten verdrängter Luft in diesen Bereich verhindert werden. Durch die Verdrängung von Luft aus dem Bereich zwischen der Membran und der Kontaktfläche wird verhindert, dass die Messung des Augendrucks, durch Lufteinschlüsse verfälscht wird.

In einer Ausführungsvariante ist die Membran von ausserhalb des Schutzüberzugs betrachtet konkav ausgebildet. Die konkave Ausgestaltung der Membran ermöglicht eine optimale Anpassung an Messkopfspitzen mit einer konkav ausgestalteten Kontaktfläche.

In einer Ausführungsvariante weist der Mantel dem Innenraum zugewandte Kanäle auf, welche sich von der Membran zur Öffnung hin erstrecken. Diese Kanäle ermöglichen das Abführen von Luft aus dem Bereich zwischen der Membran und der Kontaktfläche. Insbesondere wird die Abfuhr von verdrängter Luft ermöglicht, wenn die Messkopfspitze in den Schutzüberzug eingeführt ist und der Mantel auf der Messkopfspitze anliegt.

In einer Ausführungsvariante weist der Mantel einen abstehenden, die Öffnung umlaufenden Kragen auf und der oben genannte Haftbereich ist auf einem dem Innenraum zugewandten Teil des Kragens angeordnet. Der Kragen weist den Vorteil auf, dass er im auf der Messkopfspitze angebrachten Zustand des Schutzüberzugs Flüssigkeiten, beispielsweise Tränenflüssigkeit, auffangen kann, die beim Aufsetzen der Messkopfspitze auf ein Auge der Messkopfspitze entlang rinnen. Dadurch wird verhindert, dass ein mit der Messkopfspitze verbundener Griff glitschig wird. Zudem kann der Schutzüberzug am Kragen manuell vom Messkopf entfernt werden.

Vorzugsweise weist die Membran eine Shorehärte von weniger als 30 bis 40 auf. Dadurch wird ein gleichmässiges Anbringen des Schutzüberzugs auf die Messkopfspitze, insbesondere auf die Kontaktfläche, ermöglicht.

Die vorliegende Erfindung bezieht sich neben dem Schutzüberzug auch auf ein Verfahren zum einstückigen Herstellen des Schutzüberzugs. In einem ersten Schritt wird elastisches Material in einen Hohlraum zwischen einem äusseren Formteil und einem inneren Formteil gespritzt. Erfindungsgemäss wird in einem zweiten Schritt ein Teil des Schutzüberzugs als Membran zum Abdecken der Kontaktfläche gebildet, wobei das innere Formteil und das äussere Formteil im Bereich der zu bildenden Membran aufeinander zu bewegt werden, so dass eine Membrandicke von weniger als 55µm gebildet wird. Durch das dem Spritzgussvorgang nachfolgende Umformen, welches durch das Aufeinanderzubewegen der inneren und äusseren Formteile bewirkt wird, können elastische Schutzüberzüge mit Membrandicken unter 70µm einteilig und daher kostengünstig hergestellt werden.

### Kurze Beschreibung der Zeichnungen

Nachfolgend wird eine Ausführung der vorliegenden Erfindung anhand eines Beispieles beschrieben. Das Beispiel der Ausführung wird durch die folgenden beigelegten Figuren illustriert:
Figur 1 zeigt einen Querschnitt, der schematisch einen elastischen Schutzüberzug illustriert.
Figur 2 zeigt eine Ansicht, die schematisch den elastischen Schutzüberzug mit angedeuteten Kanälen illustriert.
Figur 3 zeigt eine Aufsicht, die schematisch den elastischen Schutzüberzug mit den Kanälen illustriert.
Figur 4a zeigt einen Querschnitt, der schematisch zwei Formteile eines Heisskanalwerkzeugs in der Position des Einspritzvorgangs illustriert.
Figur 4b zeigt einen Querschnitt, der schematisch die zwei Formteile des Heisskanalwerkzeugs in der Nennposition des Umformvorgangs illustriert.
Figur 5 zeigt einen Querschnitt, der schematisch eine Messkopfspitze eines ophthalmologischen Geräteteils mit daran angebrachtem Schutzüberzug illustriert.
Figur 6 zeigt einen Querschnitt, der schematisch die Messkopfspitze mit konkaver Kontaktfläche und daran angebrachtem Schutzüberzug illustriert.

### Wege zur Ausführung der Erfindung

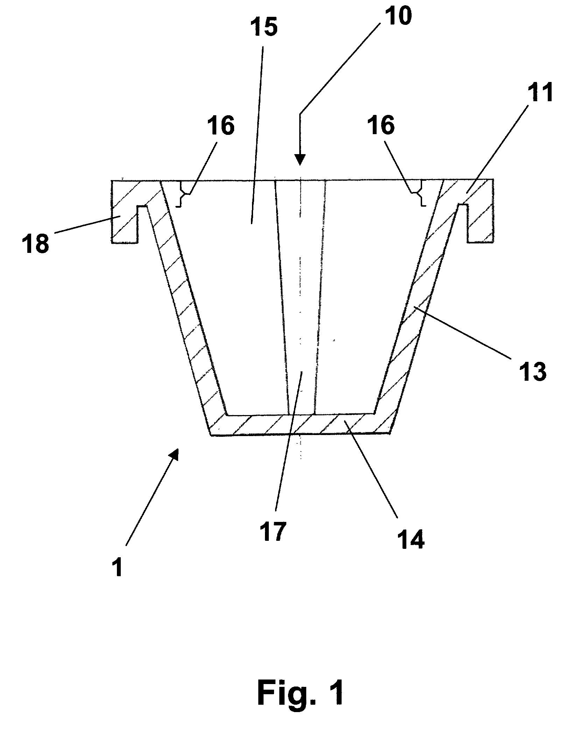

In den Figuren 1, 2, 3, 4a, 4b, 5 und 6 werden einander entsprechende, gleiche Komponenten durch gleiche Bezugszeichen bezeichnet.

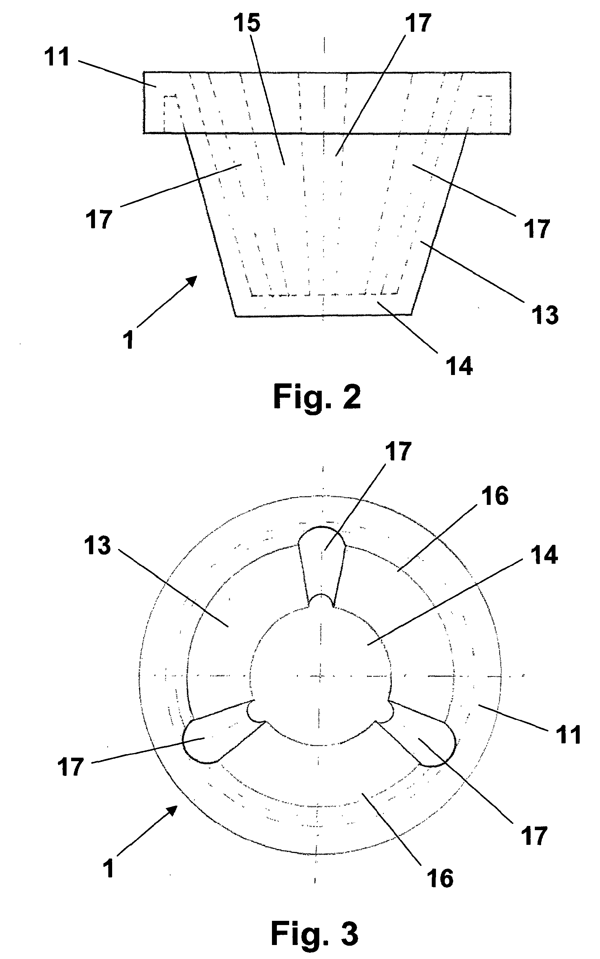

In den Figuren 1, 2, 3, 4a, 4b, 5 und 6 bezeichnet das Bezugszeichen 1 einen elastischen Schutzüberzug aus einem Elastomer, beispielsweise Silikon, Polyurethan, weiches PVC oder Kautschuk. Wie aus der Figur 1 ersichtlich ist, ist der Schutzüberzug 1 becherförmig ausgestaltet und weist einen als Membran 14 ausgestalteten kreisrunden Boden und einen mit der Membran 14 verbundenen umlaufenden Mantel 13 auf. Der Mantel 13 umfasst einen umlaufenden Kragen 11, der ein im wesentlichen U-förmiges Profil aufweist, das rund oder eckig ausgestaltet ist. Der Kragen 11 bildet einen die Öffnung 10 des Schutzüberzugs 1 umlaufenden Rand, der vom Mantel 13 in der vom Innenraum 15 abgewandten Richtung absteht. Der Durchmesser der Öffnung 10 beträgt beispielsweise 9.8mm. Die Membran 14, weist vorzugsweise beidseitig eine hochglanzpolierte, glatte Oberfläche auf. Die Membran 14 hat eine konstante Dicke im Bereiche von 40µm bis 50µm, beispielsweise 45µm. Der Durchmesser der Membran 14 beträgt beispielsweise 8mm. Der durch die Membran 14 und den Mantel 13 gebildete Innenraum 15 des Schutzüberzugs 1 verjüngt sich konisch vom Kragen 11 zur Membran 14 und ist bei planer Membran 14 im wesentlichen kegelstumpfförmig. Wie in den Figuren 2 und 3 schematisch dargestellt ist, weist der dem Innenraum 15 zugewandte Innenbereich des Mantels 13 mehrere Kanäle 17 auf, die sich jeweils gradlinig von der Membran 14 zum Kragen 11 hin erstrecken. Obwohl dies in den Figuren 2 und 3 nicht dargestellt ist, weist der Mantel 13 beispielsweise acht symmetrisch angeordnete Kanäle 17 auf. Der Mantel 13 hat beispielsweise eine Wandstärke, die sich von 0.5mm im Bereich des Kragens 11 kontinuierlich auf 0.1 mm im an die Membran 14 angrenzenden Bereich verjüngt. Der Übergang vom Mantel 13 zur Membran 14 ist beispielsweise gerundet, zum Beispiel mit einem Innenradius von 0.3mm und einem Aussenradius von 0.35mm. Der Schutzüberzug 1 weist eine Höhe, von der Membran 14 bis zur Öffnung 10, von beispielsweise 10mm auf.

Der dem Innenraum 15 zugewandte Innenbereich des Kragens 11 weist einen hochglanzpolierten, glatten Haftbereich 16 auf. Der Haftbereich 16 weist beispielsweise umlaufend eine Breite von 0.5 bis 2mm auf. Ausser an den oben erwähnten Stellen, nämlich den beiden Seiten der Membran 14 und dem Haftbereich 16, weist der Schutzüberzug 1 eine durch Erosionsstrukturen aufgerauhte Oberfläche auf.

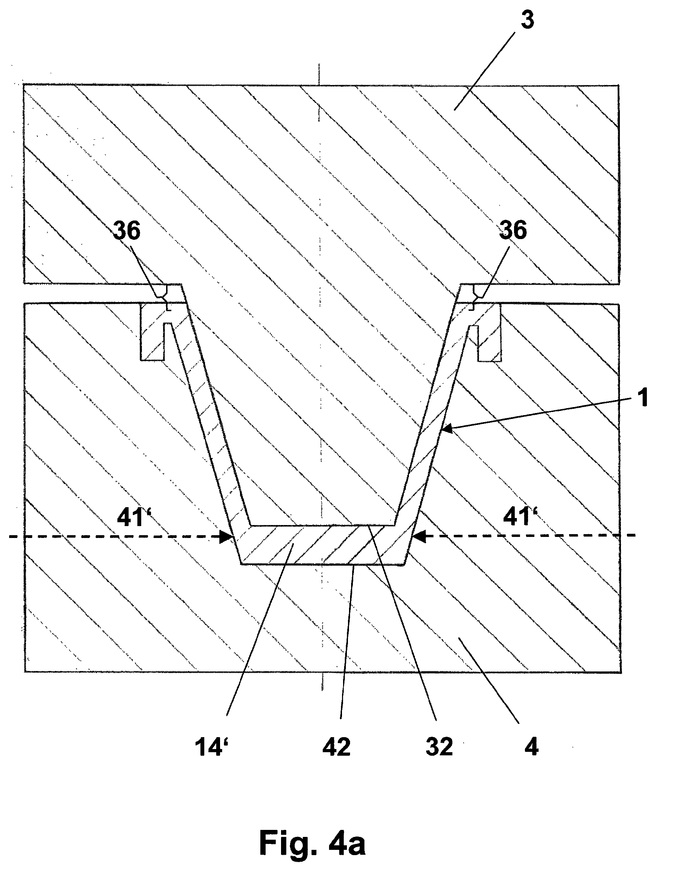

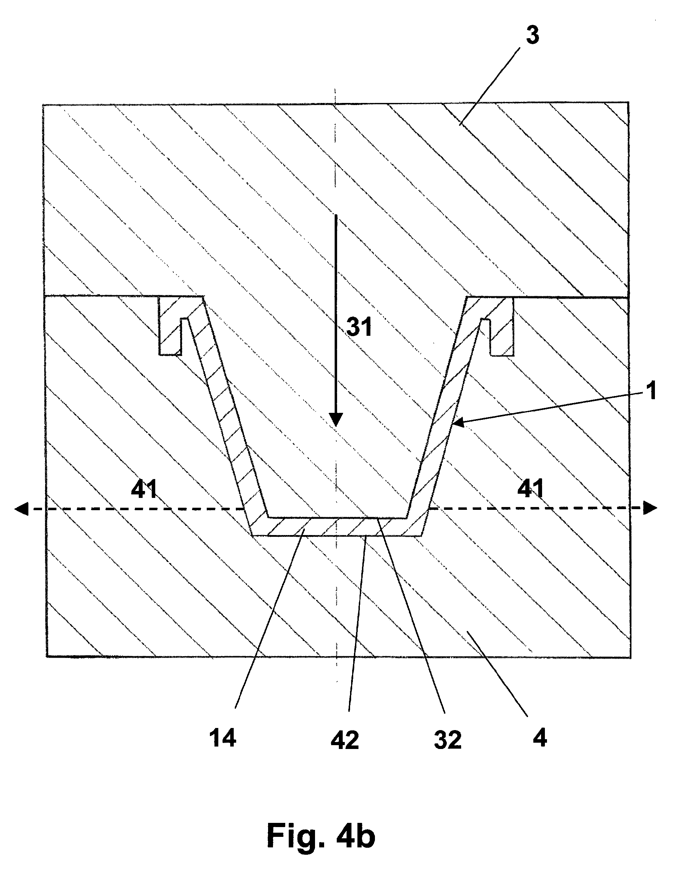

Der Schutzüberzug 1 wird mittels eines Heisskanalwerkzeugs einstückig hergestellt. Der Schutzüberzug 1 wird einstückig im Spritzgussverfahren mit anschliessender Umformung hergestellt. Die Oberflächenbeschaffenheit des Schutzüberzugs 1 wird durch entsprechende Oberflächenstrukturen der verwendeten Formteile 3, 4 (siehe Figur 4a) des Heisskanalwerkzeugs bestimmt. Die entsprechenden Oberflächenbereiche 32 und 42 respektive 36 der Formteile 3, 4 zur Bildung der Membran 14 und des Haftbereichs 16 sind hochglanzpoliert. Wie in der Figur 4a durch die Pfeile 41' schematisch dargestellt ist, wird in einem ersten Schritt das flüssige elastische Material im Membranbereich in den Hohlraum zwischen den Formteilen 3, 4 eingespritzt. Im Moment des Einspritzens befindet sich der durch das (innere) Formteil 3 repräsentierte Kern im Innern des (äusseren) Formteils 4 nicht in der Nennposition der Membrandicke sondern beispielsweise etwa 300µm davor. In dieser Einspritzstellung haben die Einspritzöffnungen freien Zugang zum Membranbereich. Auf die Bildung des Kragens 11 wird hier nicht näher eingegangen, da die Herstellung von Strukturen mit solchen Wandstärken mit herkömmlichen Spritzgussverfahren möglich ist. Nach dem Einspritzen des elastischen Materials, wenn der Hohlraum zwischen den Formteilen 3, 4 gefüllt ist, wird das innere Formteil 3, wie durch den Pfeil 31 angedeutet, in die Nennposition gebracht, so dass die Oberflächenbereiche 32 und 42 zur Bildung einer Membrandicke von 50µm einen Abstand von 50µm haben. Wie in der Figur 4b durch die Pfeile 41 schematisch dargestellt wird, fliesst das elastische Material, das dabei verdrängt wird, ab. Die dabei entstehenden Anschlussstellen, die sich ausserhalb des Membranbereichs befinden, werden nach der Aushärtung des elastischen Materials mechanisch entfernt. Auf die Verdrängung von Überschussmaterial im Kragenbereich wird hier nicht eingegangen.

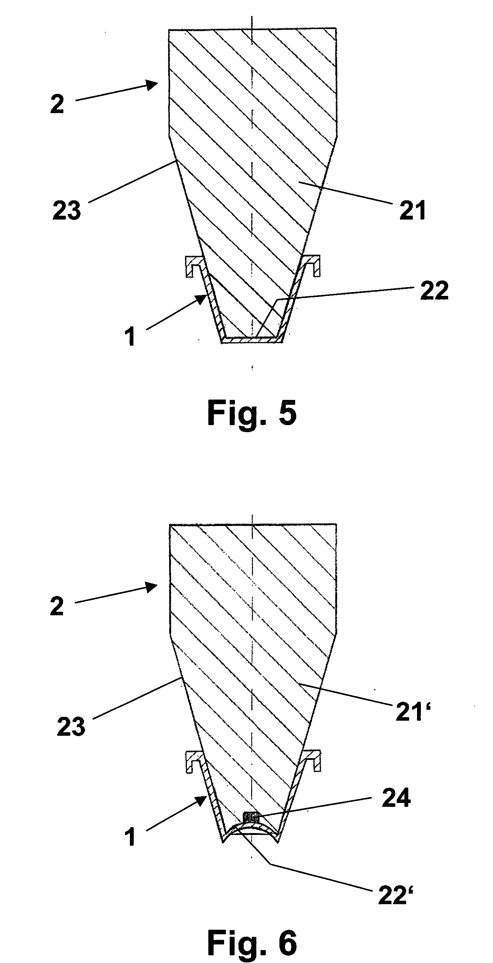

Zum Anbringen des Schutzüberzugs 1 auf die Messkopfspitze 21 des ophthalmologischen Geräteteils 2 wird die Messkopfspitze 21 durch die Öffnung 10 in den Innenraum 15 des sterilen Schutzüberzugs 1 eingeführt (Figuren 5 und 6). Beim Einführen kommt die dem Innenraum 15 zugewandte Seite der Membran 14 in Kontakt mit der Kontaktfläche 22 der Messkopfspitze 21. Durch Weiterführen der Einführbewegung wird der Aussenbereich 23 der Messkopfspitze 21 an den Haftbereich 16 des Kragens 11 angedrückt, wodurch eine Haftung des Haftbereichs 16 am Aussenbereich 23 der Messkopfspitze 21 bewirkt wird. Luft, die zwischen der Membran 14 und der Kontaktfläche 22 eingeschlossen ist kann beispielsweise mittels eines konvexen, komprimierbaren Stempels verdrängt und über den Aussenbereich 23 der Messkopfspitze 21, insbesondere über die Kanäle 17, weggeführt werden. Der Schutzüberzug 1 haftet sowohl mit dem Haftbereich 16 am Aussenbereich 23 der Messkopfspitze 21 als auch mit der Membran 14 an der Kontaktfläche 22 der Messkopfspitze 21, wie in den Figuren 5 und 6 dargestellt wird. Der Schutzüberzug 1 kann nach Gebrauch weggeworfen oder recycliert werden.

Wie in der Figur 6 dargestellt ist, eignet sich der vorgeschlagene Schutzüberzug 1 nicht nur für Messköpfe 21 mit ebenen Kontaktflächen 22, sondern auch für konkave (oder konvexe) Kontaktflächen 22'. Bei konkaven oder konvexen Kontaktflächen sollte der Krümmungsradius des Andruckbereichs eines für die Luftverdrängung verwendeten Stempels allerdings kleiner als der Krümmungsradius der Kontaktfläche 22' sein. Figur 6 zeigt insbesondere ein Beispiel, in welchem der Schutzüberzug 1 auf eine Spitze 21' eines Konturtonometers nach EP 1250884 angebracht ist, das eine konkave Kontaktfläche 22' aufweist, in die ein Drucksensor 24 mit gleicher Kontur wie die konkave Kontaktfläche 22' eingelassen ist.

Abschliessend soll angeführt werden, dass der vorgeschlagene Schutzüberzug 1 selbst bei Kontaktflächen 22 anwendbar ist, die Oberflächen mit Stufen, Absätzen und/oder Spalten aufweisen, wenn diese ohne Beeinträchtigung der Messung von einer stetigen Kurvenform überspannt werden dürfen und in dem gestuften Bereich Luft zwischen dem Schutzüberzug 1 und der Kontaktfläche 22 eingeschlossen sein darf.

## Patentansprüche

1. Schutzüberzug (1) für ophthalmologische Tonometer, welche eine Kontaktfläche (22, 22') zum Aufsetzen auf ein Auge aufweisen, **dadurch gekennzeichnet**
**dass** der Schutzüberzug (1) einstückig ist, und
**dass** der Schutzüberzug (1) eine elastische Membran (14) zum Abdecken der Kontaktfläche (22, 22') umfasst, welche Membran (14) eine Membrandicke von weniger als 55µm aufweist.

2. Schutzüberzug (1) nach Anspruch 1, **dadurch gekennzeichnet, dass** der Schutzüberzug (1) eine Öffnung (10) aufweist, dass der Schutzüberzug (1) einen die Öffnung (10) umschliessenden Mantel (13) aufweist, welcher Mantel (13) einen sich von der Öffnung (10) zur Membran (14) konisch verjüngenden Innenraum (15) bildet, und dass der Mantel (13) einen dem Innenraum (15) zugewandten Haftbereich (16) aufweist.

3. Schutzüberzug (1) nach Anspruch 2, **dadurch gekennzeichnet, dass** der Mantel (13) aus einem Elastomer, insbesondere aus Silikon, hergestellt ist, dass der Haftbereich (16) eine glatte Oberfläche aufweist, und dass der Haftbereich (16) in einem die Öffnung (10) umlaufenden Bereich angeordnet ist.

4. Schutzüberzug (1) nach einem der Ansprüche 1 bis 3, **dadurch gekennzeichnet, dass** die Membran (14) aus einem Elastomer, insbesondere aus Silikon, hergestellt ist, dass die Membran (14) eine glatte Oberfläche aufweist, welche Oberfläche im auf dem Tonometer angebrachten Zustand des Schutzüberzugs (1) auf der Kontaktfläche (22, 22') haftet.

5. Schutzüberzug (1) nach einem der Ansprüche 1 bis 4, **dadurch gekennzeichnet, dass** die Membran (14) von ausserhalb des Schutzüberzugs (1) betrachtet konkav ausgebildet ist.

6. Schutzüberzug (1) nach einem der Ansprüche 1 bis 5, **dadurch gekennzeichnet, dass** die Membran (14) eine konstante Membrandicke im Bereich von 30µm bis 50µm aufweist.

7. Schutzüberzug (1) nach einem der Ansprüche 2 bis 6, **dadurch gekennzeichnet, dass** der Mantel (13) dem Innenraum (15) zugewandte Kanäle (17) aufweist, welche sich von der Membran (14) zur Öffnung (10) hin erstrecken.

8. Schutzüberzug (1) nach einem der Ansprüche 2 bis 7, **dadurch gekennzeichnet, dass** der Mantel (13) einen abstehenden, die Öffnung (10) umlaufenden Kragen (11) aufweist, und dass der genannte Haftbereich (16) auf einem dem Innenraum (15) zugewandten Teil des Kragens (11) angeordnet ist.

9. Schutzüberzug (1) nach einem der Ansprüche 1 bis 8, **dadurch gekennzeichnet, dass** die Membran (14) eine Shorehärte von weniger als 30 bis 40 aufweist.

10. Verfahren zum Herstellen eines Schutzüberzugs (1) für ophthalmologische Tonometer, welche eine Kontaktfläche (22, 22') zum Aufsetzen auf ein Auge aufweisen, in welchem Verfahren in einem ersten Schritt elastisches Material in einen Hohlraum zwischen einem äusseren Formteil (4) und einem inneren Formteil (3) gespritzt wird, **dadurch gekennzeichnet,**
**dass** der Schutzüberzug (1) einstückig hergestellt wird, wobei in einem zweiten Schritt ein Teil des Schutzüberzugs (1) als Membran (14) zum Abdecken der Kontaktfläche (22, 22') gebildet wird, indem das innere Formteil (3) und das äussere Formteil (4) im Bereich der zu bildenden Membran (14) aufeinander zu bewegt werden, so dass eine Membrandicke von weniger als 55µm gebildet wird.

11. Verfahren nach Anspruch 10, **dadurch gekennzeichnet, dass** das innere Formteil (3) und das äussere Formteil (4) so geformt werden, dass der Schutzüberzug (1) gemäss einem der Ansprüche 1 bis 9 gebildet wird.
